**Europäisches Patentamt**

**European Patent Office**

**Office européen des brevets**

(11) Veröffentlichungsnummer: **0 040 291**
**B1**

(12) **EUROPÄISCHE PATENTSCHRIFT**

(45) Veröffentlichungstag der Patentschrift: **28.05.86**

(51) Int. Cl.⁴: **A 61 F 2/64**

(21) Anmeldenummer: **81100947.1**

(22) Anmeldetag: **11.02.81**

(54) **Prothesengelenk für Knie- und Oberschenkel-Amputierte.**

(30) Priorität: **19.05.80 DE 3019048**

(43) Veröffentlichungstag der Anmeldung:
**25.11.81 Patentblatt 81/47**

(45) Bekanntmachung des Hinweises auf die
Patenterteilung:
**28.05.86 Patentblatt 86/22**

(84) Benannte Vertragsstaaten:
**BE CH DE FR GB LI NL**

(56) Entgegenhaltungen:
**DE-C- 570 181**
**DE-C- 749 004**
**DE-C- 807 536**
**DE-C- 831 871**
**DE-U-8 013 429**
**FR-A-1 046 195**
**US-A-2 883 982**

(73) Patentinhaber: **Sanitätshaus Günter Munny
GmbH
Paffrather Strasse 15
D-5060 Bergisch Gladbach 2 (DE)**

(72) Erfinder: **Munny, Günter
Wipperfürther Strasse 49
D-5064 Odenthal-Eikamp (DE)**

(74) Vertreter: **Patentanwälte Dipl.-Ing. W. Dahlke
Dipl.-Ing. H.-J. Lippert
Frankenforster Strasse 137
D-5060 Bergisch Gladbach 1 (DE)**

## Beschreibung

Die Erfindung betrifft ein Prothesengelenk für Knie- und Oberschenkel-Amputierte, das aus einem hohlen, oberseitig offenen Schaft zur Aufnahme des Oberschenkelstumpfes und einem mit diesem Schaft gelenkig verbundenen Ansatzstück zur Befestigung einer künstlichen Wade mit Fußteil besteht.

Der Schaft wird mit Hilfe eines Abgusses des intakten Oberschenkelstumpfes hergestellt und wird dann beim Gebrauch über diesen Stumpf geschoben und mit Hilfe von Halteriemen oder dergleichen in an sich bekannter Weise fest mit diesem verbunden. In entsprechender Weise wird an dem Ansatzstück ein Rohradapter befestigt, der mit einer sogenannten Kosmetik aus Schaumstoff überzogen ist.

Die bisher für diese Zwecke verwendeten Prothesengelenke sind einerseits zu schwer und somit lästig zu tragen. Andererseits haben die bekannten Prothesengelenke den Nachteil, daß man beim Abwinkeln des künstlichen Unterschenkels deutlich erkennen kann, daß es sich um eine Prothese handelt. Beim Abwinkeln dieser bekannten Prothesengelenke werden Längendifferenzen zwischen einem gesunden Bein und der Prothese sichtbar, und es treten vorn an der Stelle, an der beim natürlichen Bein die Kniescheibe sitzt, scharfe Kanten bzw. Vorsprünge heraus.

Durch die DE—U—8 013 429 ist ein solches Prothesengelenk bekanntgeworden, bei dem die Gelenkverbindung ein fest mit dem Ansatzstück verbundenes Führungsstück mit geradlinigen Führungsnuten, ein durch dieses verschiebbar geführtes Gleitstück, einen durch dieses mittels kreisbogenförmiger Führungsleisten verschiebbar geführten Träger, der fest mit dem Schaft verbunden ist, und ein zwischen dem kreisbogenförmigen Träger einerseits und dem Führungsstück andererseits angeordnetes Getriebe umfaßt, das beim Verschwenken des Schaftes gegenüber dem Ansatzstück ein Vorschieben des Gleitstückes und damit des Schaftes bewirkt.

Auch bei diesem bekannten Prothesengelenk erfolgt beim Verschwenken des Ansatzstückes gegenüber dem Schaft ein entsprechendes Vorschieben bzw. bei umgekehrter Schwenkrichtung ein Zurückschieben des Gleitstückes und damit des Schaftes. Diese beiden Bewegungen sind aber dort nicht streng proportional zueinander, was gewisse Nachteile bei der Benutzung der Prothese mit sich bringt. Außerdem bewirkt eine auch nur geringe Lose in den beiden Anlenkpunkten des Lenkers bei dem bekannten Prothesengelenk je nach Stellung desselben eine nicht ganz einwandfreie Kopplung der beiden Bewegungen.

Der Ersatz des Lenkers durch ein einfaches Zahnradgetriebe erschien nicht möglich, obgleich mit dem Prothesengelenk nach der DE—C—570 181 Versuche damit angestellt wurden. Auch dort ließen sich aber die Schwierigkeiten bei der Realisierung offensichtlich nicht ganz beseitigen, und man sah sich gezwungen, zusätzlich eine Reibungsbremse vorzusehen, die bei Gewichtsbelastung der Prothese, entgegen der Wirkung einer Feder, wirksam wurde.

Aus der genannten Druckschrift konnte keine Anregung für eine vernünftige und voll wirksame Lösung der Aufgabe gefunden werden. Diese der Erfindung zugrundeliegende Aufgabe besteht darin, ein verbessertes Prothesengelenk für Knie- und Oberschenkel-Amputierte zu schaffen, das die vorgenannten Nachteile nicht besitzt, also trotz geringen Gewichts stabil ist, dynamischer in der Gangphase ist und eine bessere sog. Kosmetik ermöglicht.

Diese Aufgabe wird erfindungsgemäß dadurch gelöst, daß das Getriebe eine am Träger befestigte kreisbogenförmige Zahnstange, ein mit dieser kämmendes, drehbar im Gleitstück gelagertes Zahnritzel und eine mit diesem kämmende, am Führungsstück befestigte geradlinige Zahnstange umfaßt. Dieses Getriebe bewirkt beim Verschwenken des Schaftes gegenüber dem Ansatzstück ein mit der Verschwenkbewegung synchrones Vorschieben des Gleitstückes und damit des Schaftes.

Für den Fall, daß der durch ein Verschwenken des Schaftes um 90° gegenüber dem künstlichen Unterschenkel hervorgerufene Vorschub des Gleitstückes zu klein oder zu groß ist, wird vorgeschlagen, daß das Zahnritzel zwei nebeneinander und koaxial angeordnete Zahnräder mit unterschiedlichen Teilkreisdurchmessern aufweist, deren eines mit der kreisbogenförmigen Zahnstange am Träger und deren anderes mit der geradlinigen Zahnstange am Führungsstück kämmt. Auf diese Weise läßt sich in einfacher Weise eine Untersetzung oder Übersetzung erzielen und der anatomisch richtige Vorschub des Gleitstückes und damit des Schaftes realisieren.

Vorzugsweise besteht dabei das eine der beiden Zahnräder aus zwei Hälften, die beiderseits des anderen Zahnrades angeordnet sind. Dabei sind dann den beiden Zahnradhälften zwei Zahnstangen zugeordnet. Durch diese Anordnung wird eine gleichmäßige, symmetrische Kräfteverteilung erzielt.

Um zu verhindern, daß sich das den künstlichen Unterschenkel tragende Ansatzstück im Prothesengelenk vom Schaft und damit vom Oberschenkelstumpf löst, können alle bekannten Haltemittel verwendet werden. Vorzugsweise ist jedoch vorgesehen, daß der am Schaft befestigte Träger unterseitig beiderseits C-förmig vom Gleitstück umfaßt wird.

In entsprechender Weise wird vorgeschlagen, daß in dem mit dem künstlichen Unterschenkel verbundenen Führungsstück Nuten vorgesehen sind, in die entsprechende, nach außen vorstehende Führungsleisten des Gleitstückes eingreifen, so daß diese Führungsleisten ebenfalls C-förmig umfaßt, werden. Ein unbeabsichtigtes Lösen eines derart ausgebildeten Gelenks ist also nicht möglich.

Vorzugsweise ist weiterhin vorgesehen, daß das Gleitstück und/oder des Führungsstück und/ oder der Träger aus Werkstoffen mit geringen gegenseitigen Reibungswerten, vorzugsweise Kunststoffen, bestehen. In diesem Falle kann man dann auf eine besondere Schmierung verzichten, die sonst Probleme bezüglich einer Verschmutzung des Hosenbeins mit sich bringen würde.

Um die Schwenkbewegung des Prothesengelenks zu begrenzen, sind am vorderen und am hinteren ende des Führungsstücks Anschläge befestigt.

Außerdem ist ein das Prothesengelenk vorderseitig abdeckendes elastisches Band vorgesehen, das oberseitig am Schaft und unterseitig am Ansatzstück befestigt ist und diese beiden Teile über eine größere Länge hinweg überdeckt, vor allem aber die Bestandteile des Prothesengelenkes abdeckt.

Der künstliche Unterschenkel kann in an sich bekannter Weise mit dem Ansatzstück verbunden werden. Vorzugsweise ist dort zum einstellbaren Anbringen ein Haltedorn vorgesehen, der sich nach unten konisch erweitert und mit Hilfe an sich bekannter Gegenstücke eine Ausrichtung und Verstellung des künstlichen Unterschenkels in gewissem Umfang ermöglicht.

Eine weitere Verstellung in seitlicher Richtung ist dadurch möglich, daß im Führungsstück eine Reihe von mehreren, in gleichmäßigen Abständen angeordneten Reihen von Gewindebohrungen vorgesehen sind. Man kann dann das Ansatzstück je nach den statischen Verhältnissen mehr oder weniger seitlich versetzt anschrauben.

Bei Herstellung des Führungsstücks aus Kunststoff wird eine sichere Halterung ohne Lockerung der Schraubverbindung durch eine in das Führungsstück eingelassene metallische Verstärkungsplatte sichergestellt, die die Gewindebohrungen enthält.

Um schließlich auch eine sichere Verbindung zwischen dem Schaft und dem Oberschenkelstumpf zu erzielen; sind am oberen Rand des Schaftes auf seiner Außenseite mehrere Rillen vorgesehen.

Die Erfindung wird nachstehend in zwei Ausführungsbeispielen anhand der Zeichnung näher erläutert. Dabei zeigen:

Fig. 1 eine Seitenansicht eines erfindungsgemäßen Prothesengelenks in gestrecktem Zustand;

Fig. 2 eine Seitenansicht des Prothesengelenks nach Fig. 1, jedoch im abgewinkelten Zustand;

Fig. 3 einen Schnitt längs der Linie III—III in Fig. 1 und

Fig. 4 einen Schnitt ähnlich Fig. 3 durch eine abgewandelte Ausführungsform.

Wie man aus der Zeichnung erkennt, besteht das Prothesengelenk aus einem hohlen, oberseitig offenen Schaft 1, der auf den Oberschenkelstumpf aufgeschoben und mit diesem in an sich bekannter Weise fest verbunden wird. Die Verbindung wird durch die in den Schaft eingebrachten Halterillen 24 noch verbessert. Der Schaft besteht aus Kunststoff und wurde vorher nach einem Abguß des Oberschenkelstumpfes angefertigt.

Das Gelenk umfaßt ferner ein Ansatzstück 3, das aus einer Halteplatte und einem unterseitig einstückig damit verbundenen Haltedorn 4 besteht, der eine einstellbare Befestigung des künstlichen Unterschenkels 2 gestattet.

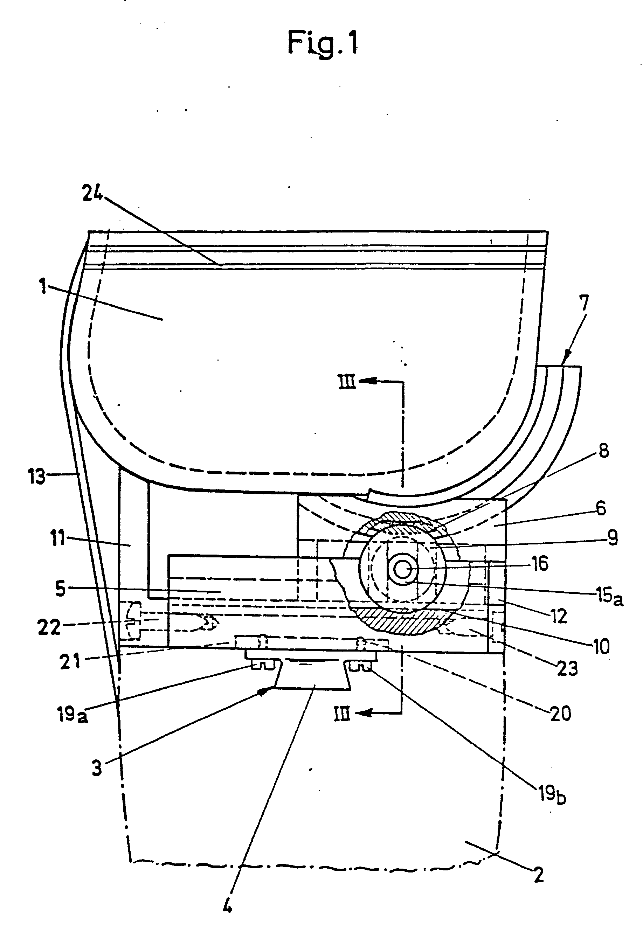

Das Prothesengelenk umfaßt ferner eine Gelenkverbindung zwischen dem Schaft 1 und dem Ansatzstück 3. Diese Gelenkverbindung weist ein fest mit dem Ansatzstück verbundenes Führungsstück 5 auf, das geradlinige Führungsnuten 17 besitzt. Das Ansatzstück 3 ist, wie aus Fig. 1 zu ersehen ist, mittels Befestigungsschrauben 19a und 19b mit dem Führungsstück verschraubt. Die Schrauben werden dabei in diejenigen Gewindebohrungen 20 in einer in das Führungsstück eingelassenen Verstärkungsplatte 21 aus Metall eingeschraubt, die dem Ansatzstück 3 die richtige Lage geben.

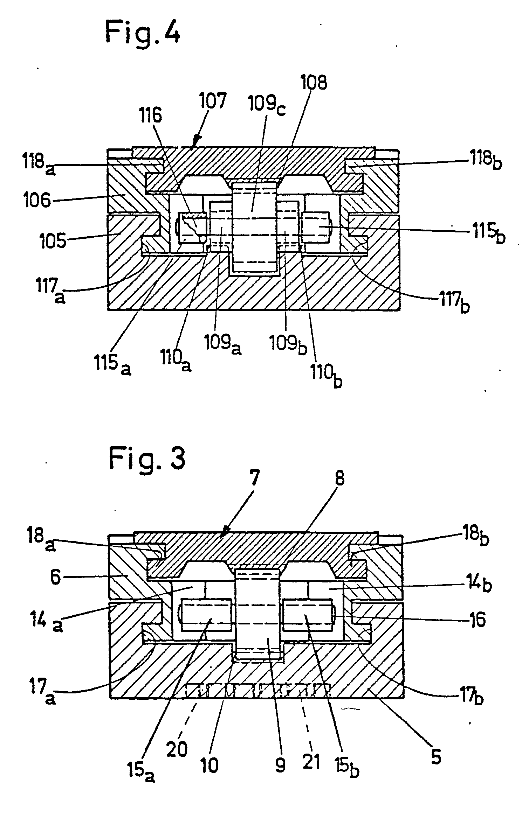

Wie man aus Fig. 1 in Verbindung mit Fig. 3 erkennt, weist die Gelenkverbindung ferner einen kreisbogenförmigen Träger 7 mit seitlich vorstehenden kreisförmigen Führungsleisten 18a und 18b auf. Deiser Träger 7 ist am unteren Ende des Schaftes 1 hinten befestigt. Wie man aus Fig. 3 erkennt, werden die besagten Führungsleisten C-förmig von dem entsprechend mit einer Nut versehenen Gleitstück 6 umgriffen.

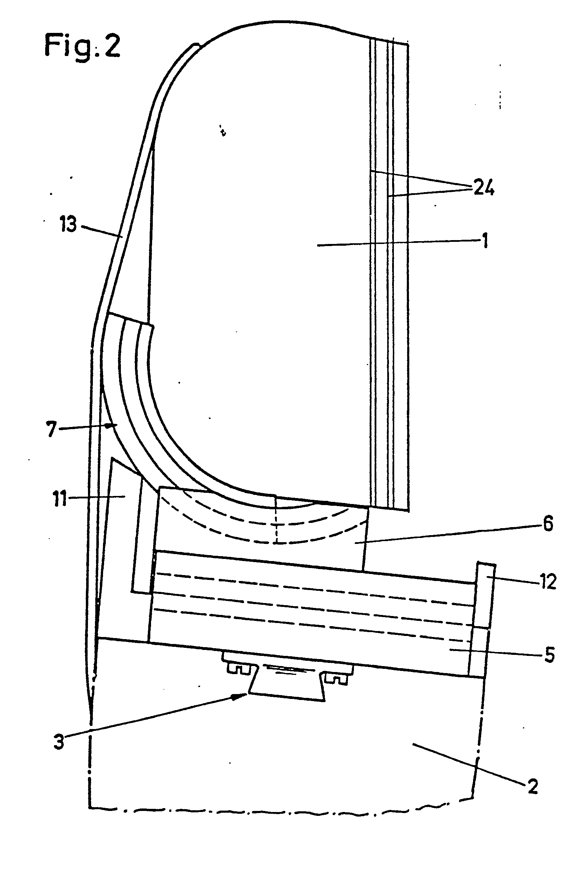

Wenn das Führungsstück 5 starr mit dem Gleitstück 6 verbunden wäre, wäre nur eine einfache Schwenkbewegung des Prothesengelenks möglich. Um zusätzlich eine erwünschte Vorschubbewegung zu erzielen, ist das Gleitstück unterseitig mit nach außen vorstehenden Führungsleisten versehen, die in entsprechende Führungsnuten 17a und 17b im Führungsstück 5 eingreifen. Diese Führungsleisten und Führungsnuten sind geradlinig ausgebildet und gestatten eine geradlinige Verschiebung des Gleitstückes 6 gegenüber dem Führungsstück 5, wie durch Vergleich der Figuren 1 und 2 zu erkennen ist.

Zur zwangsläufigen Zuordnung der Vorschubbewegung zur Schwenkbewegung dient ein Zahnradgetriebe, das zwischen den vorgenannten Teilen angeordnet ist. Im Ausführungsbeispiel gemäß Figuren 1 bis 3 besteht dieses Getriebe aus einer kreisbogenförmigen Zahnstange 8 im Träger 7, und zwar in der Mitte zwischen den beiden von diesem sietlich vorstehenden kreisförmigen Führungsleisten 18a und 18b.

Das Zahnradgetriebe besteht ferner aus einem Zahnritzel 9, das drehbar auf einer festen Achse 16 sitzt, die über Klemmhülsen 15a und 15b aus Kunststoff in entsprechende Halteschlitze 14a und 14b im Gleitstück 6 eingeklemmt ist. Das Zahnritzel kämmt oberseitig mit der kreisbogenförmigen Zahnstange 8 des Trägers 7, andererseits aber auch mit einer geradlinigen Zahnstange 10, die sich im Führungsstück 5, zwischen dessen beiden Führungsnuten 17a und 17b, befindet.

Diese Anordnung hat zur Folge, daß bei einer Verschwenkung des Schaftes 1 aus der in Fig. 1 dargestellten Lage in die in Fig. 2 dargestellte Lage die kreisbogenförmige Zahnstange 8 das Zahnritzel 9 dreht, so daß dieses zwangsläufig auf der geradlinigen Zahnstange 10 des Führungsstücks 5 abrollen muß. Das hat zur Folge, daß das Zahnritzel 9 seine Welle 16 und damit das mit dieser Welle verbundene Gleitstück 6 in der gleichen Richtung mitnimmt. Da das Gleitstück 6 aber in seinem oberen Teil beiderseits kreisbogenförmige Führungsnuten aufweist, in denen sich die kreisbogenförmigen Führungsleisten 18a und 18b des Trägers 7 befinden, wird zwangsläufig auch dieser Träger 7 von der Vorschubbewegung erfaßt und bewegt damit den Schaft 1 nach vorn bzw., wenn man die Lage des Schaftes 1, der mit dem Oberschenkelstumpf verfunden ist, als ortsfest annehmen will, erzwingt eine Rückbewegung des künstlichen Unterschenkels 2. Jedenfalls hat diese durch das erfindungsgemäße Prothesengelenk erzwungene Dreh-Gleit-Bewegung zur Folge, daß sich der künstliche Unterschenkel gegenüber dem Oberschenkelstumpf praktisch in derselben Weise bei einer Verschwenkung bewegt, wie bei einem unversehrten Kniegelenk.

Am vorderen und hinteren Ende des Führungsstücks sind Anschläge 11 und 12 befestigt, die die Bewegung des Gleitstücks 6 und damit auch die Schwenkbewegung zwischen dem Schaft 1 und dem künstlichen Unterschenkel 2 nach beiden Seiten begrenzen. Diese Anschläge sind durch Befestigungsschrauben 22 bzw. 23 am Führungsstück befestigt.

Um nach vorn einen völlig glatten Abschluß zu erzielen, ist das Gelenk durch ein elastisches Band 13 abgedeckt, das an seinen beiden Enden fest mit dem Schaft bzw. dem Ansatzstück verbunden ist und den Spalt zwischen diesen beiden Teilen abdeckt.

Bei der in Fig. 4 dargestellten abgewandelten Ausführungsform ist ein Zahnradgetriebe mit einer anderen Übersetzung vorgesehen. Dieses Zahnradgetriebe umfaßt eine kreisbogenförmige Zahnstange 108 im Träger 107, ein mit dieser Zahnstange kämmendes Zahnrad 109c, das lose auf einer festen Achse 116 sitzt, die wieder durch Klemmhülsen 115a und 115b in entsprechenden Halteschlitzen des Gleitstücks 106 angeordnet und mit diesem Gleitstück fest verbunden ist. Beiderseits dieses Zahnrades 109c befinden sich zwei weitere Zahnräder 109a und 109b, die ebenfalls frei drehbar auf der Welle 116 angeordnet, aber drehfest mit dem mittleren Zahnrad 109c verbunden sind. Man kann die drei Zahnräder selbstverständlich auch aus einem Stück herstellen.

Während nun das mittlere Zahnrad 109c ausschließlich mit der oberen, kreisbogenförmigen Zahnstange 108 kämmt, unterseitig jedoch frei läuft, kämmen die beiden seitlich von diesem mitlaufenden kleineren Zahnräder 109a und 109b unterseitig mit zwei geradlinigen Zahnstangen 110a und 110b im Führungsstück 105. Es erfolgt also jetzt eine Übersetzung der Bewegungen, die sich aus den verschiedenen Teilkreisdurchmessern des mittleren Zahnrades 109c einerseits und der beiden seitlichen Zahnräder 109a und 109b andererseits ergibt.

Auch hier greifen wieder kreisförmige Führungsleisten 118a und 118b am Gleitstück 106 in entsprechend geformte Führungsnuten im Träger 107, während andererseits geradlinige Führungsnuten 117a und 117b im Führungsstück 105 die entsprechenden Führungleisten, die unterseitig nach außen vom Gleitstück 106 vorstehen, aufnehmen.

Es wird noch bemerkt, daß das erfindungsgemäße Prothesengelenk für Knie- und Oberschenkel-Amputierte mit verschiedenen Oberschenkelgrößen verwendbar ist, indem der Schaft in verschiedenen Größen hergestellt werden kann.

**Patentansprüche**

1. Prothesengelenk für Knie- und Oberschenkel-Amputierte, bestehend aus einem hohlen, oberseitig offenen Schaft (1) zur Aufnahme eines Oberschenkelstumpfes und einem mit diesem Schaft gelenkig verbundenen Ansatzstück (3) zur Befestigung eines künstlichen Unterschenkels (2), wobei die Gelenkverbindung ein fest mit dem Ansatzstück (3) verbundenes Führungsstück (5, 105) mit geradlinigen Führungsnuten (17a, 17b; 117a, 117b), ein durch dieses verschiebbar geführtes Gleitstück (6, 106), einen durch dieses mittels kreisbogenförmiger Führungsleisten (18a, 18b; 118a, 118b) verschiebbar geführten Träger (7, 107), der fest mit dem Schaft (1) verbunden ist, und ein zwischen dem kreisbogenförmigen Träger (7, 107) einerseits und dem Führungsstück (5, 105) andererseits angeordnetes Getriebe umfaßt, das beim Verschwenken des Schaftes (1) gegenüber dem Ansatzstück (3) ein Vorschieben des Gleitstückes (6, 106) und damit des Schaftes (1) bewirkt, dadurch gekennzeichnet, daß das Getriebe eine am Träger (7, 107) befestigte kreisbogenförmige Zahnstange (8, 108), ein mit dieser kämmendes, drehbar im Gleitstück (6, 106) gelagertes Zahnritzel (9, 109) und eine mit diesem kämmende, am Führungsstück (5, 105) befestigte geradlinige Zahnstange (10; 110a, 110b) umfaßt.

2. Prothesengelenk nach Anspruch 1, dadurch gekennzeichnet, daß das Zahnritzel (109) zwei nebeneinander und koaxial angeordnete Zahnräder unterschiedlicher Teilkreisdurchmesser aufweist, deren eines (109c) mit der kreisbogenförmigen Zahstange (108) am Träger (107) und deren anderes (109a) mit der geradlinigen Zahnstange (110a) am Führungsstück (105) kämmt.

3. Prothesengelenk nach Anspruch 2, dadurch gekennzeichnet, daß das eine der beiden Zahnräder aus zwei Hälften (109a, 109b) besteht, die beiderseits des anderen Zahnrades (109c) angeordnet sind, und daß diesen Hälften zwei Zahnstangen (110a, 110b) zugeordnet sind.

4. Prothesengelenk nach Anspruch 1 bis 3, dadurch gekennzeichnet, daß der am Schaft (1) befestigte Träger (7, 107) unterseitig beiderseits C-förmig vom Gleitstück (6, 106) umfaßt wird.

5. Prothesengelenk nach Anspruch 1 bis 4, dadurch gekennzeichnet, daß das Gleitstück (6, 106) und/oder das Führungsstück (5, 105) und/oder der Träger (7, 107) aus Werkstoffen mit geringen gegenseitigen Reibungswerten, vorzugsweise Kunststoffen, bestehen.

6. Prothesengelenk nach Anspruch 1 bis 5, gekennzeichnet durch am vorderen und hinteren Ende des Führungsstückes (5, 105) befestigte Anschläge (11, 12) zur Begrenzung der Schwenkbewegung des Prothesengelenks.

7. Prothesengelenk nach Anspruch 1 bis 6, gekennzeichnet durch ein das Prothesengelenk vorderseitig abdeckendes elastisches Band (13), das oberseitig am Schaft (1) und unterseitig am Ansatzstück (3) befestigt ist.

8. Prothesengelenk nach Anspruch 1 bis 7, dadurch gekennzeichnet, daß das Ansatzstück (3) unterseitig einen sich nach unten konisch erweiternden Haltedorn (4) zum einstellbaren Anbringen des künstlichen Unterschenkels (2) aufweist.

9. Prothesengelenk nach Anspruch 1 bis 8, gekennzeichnet durch eine Reihe von mehreren, in gleichmäßigen Abständen angeordneten Paaren von Gewindebohrungen (20) im Führungsstück (5, 105).

10. Prothesengelenk nach Anspruch 9, gekennzeichnet durch eine unterseitig in das Führungsstück (5) eingelassene metallische Verstärkungsplatte (21) zur Aufnahme der Gewindebohrungen (20).

11. Prothesengelenk nach Anspruch 1 bis 10, gekennzeichnet durch Rillen (24) am oberen Rand des Schaftes (1) auf dessen Außenseite.

## Revendications

1. Articulation de prothèse pour amputés du genou et de la cuisse, se composant d'une tige creuse (1), ouverte du côté supérieur, pour recevoir le moignon de cuisse, et d'une pièce à épaulement (3), reliée de manière articulée à cette tige, pour la fixation d'une jambe artificielle (2), la liaison articulée comprenant une pièce de guidage (5, 105) fixée rigidement à la pièce à épaulement (3) et comportant des rainures de guidage (17a, 17b; 117a, 117b), une pièce coulissante (6, 106) guidée dans ses déplacements par cette pièce, un support (7, 107) qui est fixé rigidement à la tige (1) et est guidé dans ses déplacements par cette pièce coulissante au moyen de nervures de guidage en arc de cercle (18a, 18b; 118a, 118b) et un engrenage disposé entre le support en arc de cercle (7, 107) d'une part et la pièce de guidage (5, 105) d'autre part, engrenage qui produit une avance de la pièce coulissante (6, 106) et, par suite, de la tige (1) lorsque la tige (1) pivote par rapport à la pièce à épaulement (3), caractérisée en ce que l'engrenage comprend une crémaillère en arc de cercle (8, 108) fixée au support (7, 107), un pignon (9, 109) en prise avec celle-ci et monté à rotation dans la pièce coulissante (8, 108) et une crémaillère rectiligne (10, 110a, 110b) en prise avec ce pignon et fixée à la pièce de guidage (5, 105).

2. Articulation de prothèse selon la revendication 1, caractérisée en ce que la pignon (109) comporte deux roues dentées de diamètres primitifs de référence différents, disposées côte à côte et coaxialement, roues dont l'une (109c) est en prise avec la crémaillère en arc de cercle (108) sur le support (107) et dont l'autre (109a) est en prise avec la crémaillère rectiligne (110a) sur la pièce de guidage (105).

3. Articulation de prothèse selon la revendication 2, caractérisée en ce que l'une des deux roues dentées se compose de deux moitiés (109a, 109b) qui sont disposées de part et d'autre de l'autre roue dentée (109c), et en ce que deux crémaillères (110a, 110b) sont associées à ces moitiés.

4. Articulation de prothèse selon l'une quelconque des revendications 1 à 3, caractérisée en ce que le support (7, 107) fixé à la tige (1) est entouré de part et d'autre en forme de C, du côté inférieur, par la pièce coulissante (6, 106).

5. Articulation de prothèse selon l'une quelconque des revendications 1 à 4, caractérisée en ce que la pièce coulissante (6, 106) et/ou la pièce de guidage (5, 105) et/ou le support (7, 107) sont faits de matières ayant de faibles coefficients de frottement mutuel, de préférence des matières plastiques.

6. Articulation de prothèse selon l'une quelconque des revendications 1 à 5, caractérisée par des butées (11, 12) fixées aux extrémités avant et arrière de la pièce de guidage (5, 105) pour la limitation du mouvement pivotant de l'articulation de prothèse.

7. Articulation de prothèse selon l'une quelconque des revendications 1 à 6, caractérisée par une bande élastique (13) qui recouvre l'articulation de prothèse du côté antérieur et qui est fixée à la tige (1) du côté supérieur et à la pièce à épaulement (3) du côté inférieur.

8. Articulation de prothèse selon l'une quelconque des revendications 1 à 7, caractérisée en ce que la pièce à épaulement (3) comporte, du côté inférieur, un tenon de retenue (4) qui sélargit en cône vers le bas, pour le montage réglable de la jambe artificielle (2).

9. Articulation de prothèse selon l'une quelconque des revendications 1 à 8, caractérisée par une seérie de plusieurs paires, disposées à intervalles réguliers, de trous filetés (20) dans la pièce de guidage (5, 105).

10. Articulation de prothèse selon la revendication 9, caractérisée par une plaque métallique de renfort (21), encastrée dans la pièce de guidage (5), pour recevoir les trous filetés (20).

11. Articulation de prothèse selon l'une quelconque des revendications 1 à 10, caractérisée par des gorges (24) au niveau du bord supérieur de la tige (1), à l'extérieur de celle-ci.

## Claims

1. Prosthetic joint for knee and upper thigh amputees and consisting of, open at the top end, a hollow shaft (1) to accommodate the stump of an upper thigh and, articulatingly connected to this shaft, an extension piece (3) for attachment of an artificial lower thigh (2), the articulating connection having, rigidly connected to the extension piece (3) a guide piece (5, 105) having rectilinear guide slots (17a, 17b; 117a, 117b), sliding member (6, 106) guided for displacement through the said guide piece, carrier (7, 107) guided for displacement through the sliding member (6, 106) by means of arcuately shaped guide strips (18a, 18b; 118a, 118b), the carrier (7, 107) being rigidly connected to the shaft (1), and further comprising, between the arcuately shaped carrier (7, 107) on the one hand and the guide piece (5, 105) on the other, a geared transmission which, upon pivoting of the shaft (1) in respect of the extension piece (3), produces a forwards movement of the sliding member (6, 106) and thus of the shaft (1), characterized in that the geared transmission comprises, mounted on the carrier (7, 107) an arcuately shaped rack (8, 108) and, meshing therewith and mounted to be rotatable in the sliding member (6, 106), a toothed pinion (9, 109) and meshing with which there is fixed on the guide piece (5, 105) a rectilinear rack (10, 110a, 110b).

2. Prosthetic joint according to Claim 1, characterized in that the toothed pinion (109) comrises two adjacently and coaxially disposed gearwheels of differing pitch circle diameter, of which one (109c) meshes with the arcuately shaped rack (108) on the carrier (107) while the other (109a) meshes with the straight rack (110a) on the guide piece (105).

3. Prosthetic joint according to Claim 2, characterized in that one of the two gearwheels comprises two halves (109a, 109b) disposed one on either side of the other gearwheel (109c) and in that two racks (110a, 110b) are associated with these halves.

4. Prosthetic joint according to Claim 1 to 3, characterized in that the carrier (7, 107) mounted on the shaft (1) is on the underside enclosed on both sides in C-shaped fashion by the sliding member (6, 106).

5. Prosthetic joint according to Claim 1 to 4, characterized in that the sliding member (6, 106) and/or the guide piece (5, 105) and/or the carrier (7, 107) consist of materials having a low mutual friction coefficient, for example synthetic plastics materials.

6. Prosthetic joint according to Claim 1 to 5, characterized by, fixed at the front and rear end of the guide piece (5, 105), abutments (11, 12) for defining the pivoting movement of the prosthetic joint.

7. Prosthetic joint according to Claim 1 to 6, characterized by, masking the prosthetic joint at the front, an elastic strip (13) fixed at the top to the shaft (1) and at the bottom to the extension piece (3).

8. Prosthetic joint according to Claim 1 to 7, characterized in that the attachment piece (3) has at the bottom a conically downwardly widening out supporting member (4) for adjustable attachment of the artificial lower thigh (2).

9. Prosthetic joint according to Claim 1 to 8, characterized by a series of pairs of threaded bores (20) disposed at regular intervals in the guide piece (5, 105).

10. Prosthetic joint according to Claim 9, characterized by a metal reinforcing plate (21) to accommodate the threaded bores (20) and let into the underside of the guide piece (5).

11. Prosthetic joint according to Claim 1 to 10, characterized by grooves (24) on the upper edge and on the outside of the shaft (1).

# Fig.1

Fig.2

# Fig. 4

# Fig. 3